(19) Europäisches Patentamt
European Patent Office
Office européen des brevets

(11) Publication number: **0 243 122**
**A2**

# (12) EUROPEAN PATENT APPLICATION

(21) Application number: **87303419.3**

(22) Date of filing: **16.04.87**

(51) Int. Cl.[3]: **C 07 D 401/12**
**C 07 D 417/12, C 07 D 241/08**
**A 61 K 31/495**
**//C07K5/06, C07D209/20**

(30) Priority: **23.04.86 GB 8609908**
**31.12.86 GB 8631082**

(43) Date of publication of application:
**28.10.87 Bulletin 87/44**

(84) Designated Contracting States:
**AT BE CH DE ES FR GB GR IT LI LU NL SE**

(71) Applicant: **Fujisawa Pharmaceutical Co., Ltd.**
**3, Doshomachi 4-chome Higashi-ku**
**Osaka-shi, Osaka 541(JP)**

(72) Inventor: **Shimazaki, Norihiko New Life Umezono**
**Daiichi No.602Sakuramura**
**Niihari-gun Ibaraki-ken(JP)**

(72) Inventor: **Hemmi, Keiji**
**No. 2-17-1, Umezono Sakuramura**
**Niihari-gun Ibaraki-ken(JP)**

(72) Inventor: **Nakaguti, Osamu**
**No. 15-34, Nakasakurazuka 1-chome**
**Toyonaka(JP)**

(72) Inventor: **Miyazaki, Yoshio**
**No. 1-13, Hiramatsu 2-chome**
**Itami(JP)**

(72) Inventor: **Hashimoto, Masashi Kashiwa Mansion No.212**
**No. 2-11-6, Takezono Sakuramura**
**Niihari-gun Ibaraki-ken(JP)**

(74) Representative: **Pennant, Pyers et al,**
**Stevens, Hewlett & Perkins 5 Quality Court Chancery Lane**
**London, WC2A 1HZ(GB)**

(54) **Piperazine compound as PAF-antagonist.**

(57) A piperazine compound of the formula:

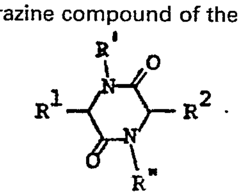

(I)

wherein R′ and R″ are each hydrogen or lower alkyl,
$R^1$ is 1-(1-methylindol-3-yl)ethyl,
1-(1-ethylindol-3-yl)ethyl,
1-(1-propylindol-3-yl)ethyl,
1-(1,2-dimethylindol-3-yl)ethyl,
1-(1-methyl-2-phenylindol-3-yl)ethyl,
1-(1,5-dimethylindol-3-yl)ethyl,
1-(5-methyl-1-propylindol-3-yl)ethyl,
1-(2,5-dimethyl-1-propylindol-3-yl)ethyl,
1-(1,2,5-trimethylindol-3-yl)ethyl
1-(1-butyl-2-methylindol-3-yl)ethyl,
1-(1-isobutylindol-3-yl)ethyl,
1-(1-methylindol-3-yl)propyl,
1-(1-ethyl-6-methylindol-3-yl)ethyl or
1-(1-ethyl-5-methylindol-3-yl)ethyl,
$R^2$ is 2-pyridylmethyl; or
$R^1$ is 1-methylindol-3-ylmethyl and
$R^2$ is 3-(or 4-)pyridylmethyl; or
$R^1$ is 1-naphthylmethyl and
$R^2$ is butyl or
$R^1$ is heterocyclic(lower)alkyl which may have one or more lower alkyl(s) on the heterocyclic ring and
$R^2$ is thiazolyl(lower)alkyl,
quinolyl(lower)alkyl,
tetahydropyridyl(lower)alkyl which may have lower alkyl on the tetrahydropyridine ring, or a group of the formula:
wherein A is lower alkylene,
$R^3$ is lower alkyl and
X is acid residue; or
$R^1$ is indolyl(lower)alkyl having lower alkyl and lower alkoxy on the indole ring, indolyl(lower)alkyl having one or more lower alkyl(s) and halogen on the indole ring, indolyl(lower)alkyl having lower alkyl and ar(lower)alkoxy on the indole ring or indolylar-(lower)alkyl having lower alkyl on the indole ring and
$R^2$ is heterocyclic(lower)alkyl,
and pharmaceutically acceptable salt thereof, which are highly potent antagonists of PAF, processes for the preparation thereof and a pharmaceutical composition comprising the same.

EP 0 243 122 A2

0243122

# PIPERAZINE COMPOUND AS PAF-ANTAGONIST

This invention relates to new PAF (Platelet Activating Factor) antagonist, piperazine compound (I) and pharmaceutically acceptable salt thereof. More particularly, it relates to a pharmaceutical composition comprising the piperazine compound (I) or pharmaceutically acceptable salt thereof as an active ingredient, and to new piperazine compound (I) and pharmaceutically acceptable salt thereof and to processes for the preparation thereof.

This invention based on new discovery by the present inventors that certain piperazine compound (I) and pharmaceutically acceptable salt thereof are highly potent antagonists of PAF and therefore useful for the prevention and treatment of diseases caused by PAF.

The piperazine compound of this invention can be represented by the following formula :

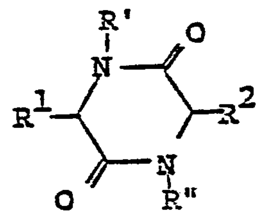

(I)

wherein R' and R" are each hydrogen or lower alkyl,

$R^1$ is 1-(1-methylindol-3-yl)ethyl,
1-(1-ethylindol-3-yl)ethyl,
1-(1-propylindol-3-yl)ethyl,
1-(1,2-dimethylindol-3-yl)ethyl,
1-(1-methyl-2-phenylindol-3-yl)ethyl,
1-(1,5-dimethylindol-3-yl)ethyl,
1-(5-methyl-1-propylindol-3-yl)ethyl,
1-(2,5-dimethyl-1-propylindol-3-yl)ethyl,
1-(1,2,5-trimethylindol-3-yl)ethyl
1-(1-butyl-2-methylindol-3-yl)ethyl,
1-(1-isobutylindol-3-yl)ethyl,
1-(1-methylindol-3-yl)propyl,
1-(1-ethyl-6-methylindol-3-yl)ethyl or
1-(1-ethyl-5-methylindol-3-yl)ethyl,

$R^2$ is 2-pyridylmethyl; or

$R^1$ is 1-methylindol-3-ylmethyl and

$R^2$ is 3-(or 4-)pyridylmethyl; or

$R^1$ is 1-naphthylmethyl and

$R^2$ is butyl; or

$R^1$ is heterocyclic(lower)alkyl which may have one or more lower alkyl(s) on the heterocyclic ring and

$R^2$ is thiazolyl(lower)alkyl,
quinolyl(lower)alkyl,
tetahydropyridyl(lower)alkyl which may have lower alkyl on the tetrahydropyridine ring, or a group of the formula :

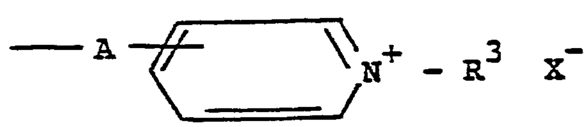

wherein A is lower alkylene,
$R^3$ is lower alkyl and
X is acid residue; or

$R^1$ is indolyl(lower)alkyl having lower alkyl and lower alkoxy on the indole ring, indolyl(lower)alkyl having one or more lower alkyl(s) and halogen on the indole ring, indolyl(lower)alkyl having lower alkyl and ar(lower)alkoxy on the indole ring or indolylar(lower)alkyl having lower alkyl on the indole ring and
$R^2$ is heterocyclic(lower)alkyl,
or pharmaceutically acceptable salt thereof.

Among the piperazine compound (I), the preferred stereochemically specific compound can be represented by the following formula :

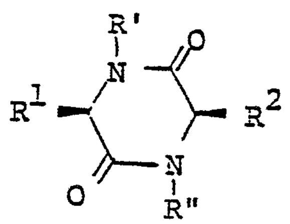

(Ia)

wherein $R^1$, $R^2$, R' and R" are each as defined above or pharmaceutically acceptable salt thereof.

The pharmaceutically acceptable salt of the compounds (I) and (Ia) may include an organic or inorganic acid addition salt such as hydrochloride, sulfate and the like.

According to this invention, the piperazine compound (I) and salts thereof can be prepared by, for example, the following processes.

Process 1 :

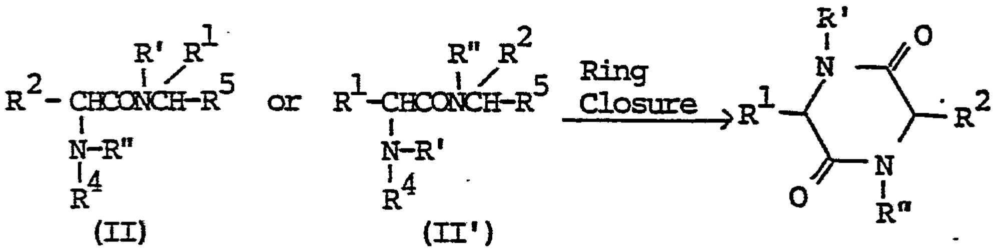


(II) (II') (I)

or their reactive derivative at the carboxy or amino group or salt thereof

or salt thereof

Process 2 :

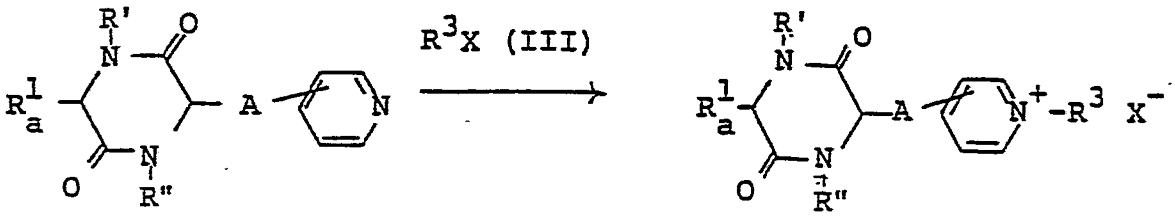


(Ib) (Ic)

or salt thereof

Process 3 :

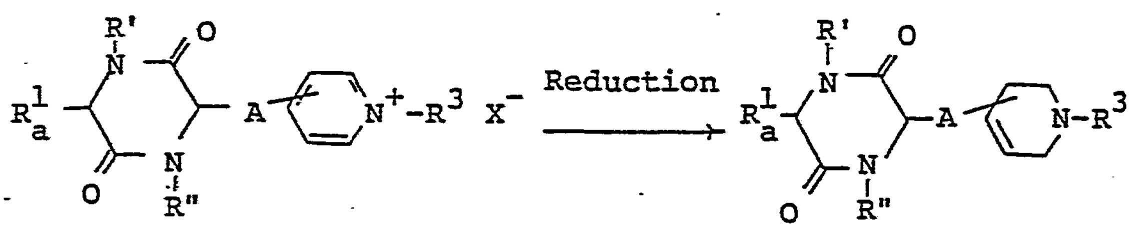


(Ic) (Id)

wherein $R^1$, $R^2$, A, $R^3$, R',R" and X are each as defined above,

$R^4$ is hydrogen or amino-protective group,

$R^5$ is carboxy or a protected carboxy and

$R^1_a$ is heterocyclic(lower)alkyl which may have one or more lower alkyl(s) on the heterocyclic ring.

The starting compounds(II)and(II') can be prepared by subjecting the corresponding two amino acids to peptide linkage formation reaction in a conventional manner.

In the above and subsequent descriptions of this specifications, suitable examples and illustrations of the various definitions are explained in detail in the followings.

The term "lower" is intended to mean 1 to 6 carbon atom(s), unless otherwise indicated.

Suitable "lower alkyl" in the terms "lower alkyl", "heterocyclic(lower)alkyl", "thiazolyl(lower)alkyl", "quinolyl(lower)alkyl", "tetrahydropyridyl(lower)alkyl" and "indolyl(lower)alkyl" may include methyl, ethyl, propyl, isopropyl, butyl, pentyl, hexyl and the like.

Suitable "ar(lower)alkyl" in the term "indolyl-ar(lower)alkyl" may include phenyl(lower)alkyl(e.g. benzyl, phenethyl, etc.) and the like.

Suitable "heterocyclic" moiety in the term "heterocyclic(lower)alkyl" may include benzene-fused heterocyclic group containing at least one hetero-atom such as oxygen, sulfur and nitrogen (e.g. indolyl, benzothienyl, etc.), heteromonocyclic group containing at least one hetero-atom such as oxygen, sulfur and nitrogen (e.g. pyridyl, thiazolyl, etc.) and the like.

Suitable "lower alkylene" may include methylene, methylmethylene, ethylene, trimethylene, tetramethylene, pentamethylene, hexamethylene and the the like.

Suitable "acid residue" may include halogen (e.g. fluorine, chlorine, bromine, iodine) and the like.

Suitable "lower alkoxy" may include methoxy, ethoxy, propoxy, isopropoxy, butoxy, pentyloxy, hexyloxy and the like.

Suitable "ar(lower)alkoxy" may include phenyl-(lower)alkoxy(e.g. benzyloxy, phenethyloxy, etc.) and the like.

"Halogen" may include fluorine, chlorine, bromine, iodine and the like.

The "amino-protective group" include a conventional amino-protective group which is used in the field of amino acid and peptide chemistry, and suitable "amino-protective group" may include acyl such as lower alkanoyl (e.g. formyl, acetyl, propionyl, etc.), aroyl (e.g. benzoyl, etc.), lower alkoxycarbonyl (e.g. methoxy-carbonyl, ethoxycarbonyl, propoxycarbonyl, t-butoxy-carbonyl, etc.), ar(lower)alkoxycarbonyl [e.g. mono(or di or tri)phenyl(lower)alkoxycarbonyl (e.g. benzyloxycarbonyl, etc.), etc.] and the like.

Suitable "protected carboxy" may include esterified carboxy such as lower alkoxycarbonyl as aforementioned, ar(lower)alkoxycarbonyl as aforementioned, and the like.

The salts of compounds (II)and(II') may include the same acid addition salt as those exemplified in the explanation of the pharmaceutically acceptable salt of the compound (I), and a salt with an inorganic or organic base and the like.

The salt of the compound (Ib) may include the same acid addition salt as those exemplified in the explanation of the pharmaceutically acceptable salt of the compound (I).

The processes as illustrated above are explained in more detail in the following.

Process 1 :

The object compound (I) or salt thereof can be prepared by subjecting the compound (II) or (II') or their reactive derivative at the carboxy or amino group or salt thereof to ring closure reaction.

Suitable reactive derivative at the amino group of the compounds (II) and (II') may include Schiff's base type imino or its tautomeric enamine type isomer formed by the reaction of the compounds (II) or (II') with a carbonyl compound such as aldehyde, ketone or the like; a silyl derivative formed by the reaction of the compounds (II) or (II') with a silyl compound such as bis(trimethylsilyl)acetamide, trimethylsilylacetamide or the like.

The suitable reactive derivative at the carboxy of compounds (II) and (II') may be a conventional one such as an acid halide (e.g. acid chloride, acid bromide, etc.), an acid azide, an acid anhydride, an activated amide, an activated ester and the like.

When free acid is used as the starting compounds (II) and (II'), the reaction may preferably be conducted in the presence of a conventional condensing agent.

The reaction can also be conducted in the presence of an organic or inorganic base such as alkali metal (e.g. sodium), alkaline earth metal (e.g. calcium), alkali or alkaline earth metal hydride (e.g. sodium hydride, calcium hydride, etc.), alkali or alkaline earth metal hydroxide (e.g. sodium hydroxide, potassium hydroxide, calcium hydroxide, etc.), alkali or alkaline earth metal carbonate or bicarbonate (e.g. sodium carbonate, potassium carbonate, sodium bicarbonate), alkali or alkaline earth metal alkoxide (e.g. sodium ethoxide, lithium methoxide, magnesium methoxide), trialkylamine (e.g. triethylamine) pyridine, bicyclodiaza compound (e.g. 1,5-diazabicyclo-[3,4,0]nonene-5, 1,5-diazabicyclo[5,4,0]undecene-5, etc.) and the like.

This reaction is usually carried out without

solvent or in the presence of a solvent which does not adversely influence the reaction such as methanol, ethanol, propanol, acetic acid, dimethylformamide, dichloroethane, toluene, benzene, xylene and the like.

The reaction temperature is not critical, and the reaction is usually carried out under heating to under cooling.

When the compound (II) or (II') having amino-protective group for $R^4$ is used as a starting compound, it may often give preferred results to remove the amino-protective group in a conventional manner (e.g. hydrolysis, catalytic reduction, etc.) prior to this ring closure reaction.

Process 2 :

The compound (Ic) can be prepared by reacting the compound (Ib) or salt thereof with the compound (III).

This reaction is usually carried out without solvent or in the presence of a solvent which does not adversely influence the reaction such as methanol, ethanol, propanol, acetic acid, dimethylformamide, dichloroethane, toluene, benzene, xylene and the like.

The reaction temperature is not critical, and the reaction is usually carried out under heating to under cooling.

Process 3 :

The compound (Id) can be prepared by reducing the compound (Ic).

In the reduction, there may usually be employed a reducing agent such as alkali metal borohydride (e.g.

sodium borohydride, etc.) and the like.

This reaction is usually carried out in a solvent which does not adversely influence the reaction such as methanol, ethanol, propanol, acetic acid, dimethylformamide, dichloroethane, toluene, benzene, xylene and the like.

The reaction temperature is not critical, and the reaction is usually carried out under heating to under cooling.

The object compound (I) and pharmaceutically acceptable salt thereof are antagonists of PAF and therefore useful as a medicine for preventing and treating diseases caused by PAF such as allergic manifestation (e.g. asthma, etc.), thrombosis, nephritis, endotoxin shock (ARDS), and the like.

The following Tests are given for the purpose of illustrating antagonism of the compound (I) against PAF.

The test compounds are listed below.

Test compound

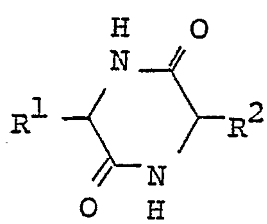

| Test compound No. | $R^1$ | $R^2$ |
|---|---|---|
| 1 | 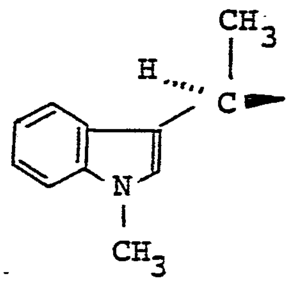 | 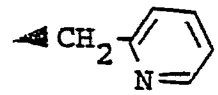 |
| 2 | 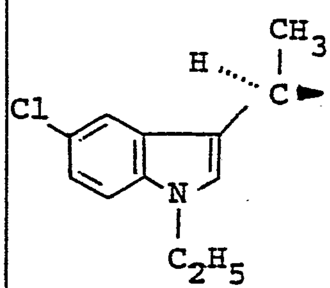 | 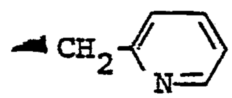 |

<u>Test 1</u> (Inhibition of platelet aggregation)

<u>Test method</u>

Blood was collected through the polyethylene catheter introduced into the carotid artery of male Japanese white rabbit (2.5 to 3 kg body weight). The blood was anticoagulated with 1 volume of 3.8% sodium citrate to 9 volume of blood. Platelet rich plasma (PRP) was prepared by centrifugation of the blood at 150 g for 10 minutes at room temperature. The prp was diluted with platelet poor plasma obtained by further centrifugation of the blood at 1,000 g for 20 minutes. The platelet number was 5 x $10^5$ cells/$mm^3$.

Platelet aggregation induced with PAF was measured by the nephelometric technique of Born and Cross [cf. Journal of Physiology 168, 178-188 (1963)] using HKK Hema tracer (trade name, made by Niko Bioscience Inc.).

Activity of inhibitor was expressed as $IC_{50}$ value, i.e. concentration required to inhibit the platelet aggregation response by 50%. The final concentration of PAF was usually 20 nM. The test result is shown in the following table.

Test result

| Test compound No. | $IC_{50}$ (μg/mℓ) |
|---|---|
| 1 | 0.16 |
| 2 | 0.015 |

Test 2 (Inhibition of hypotensive effect induced by intravenously injected PAF in rats)

Test method

Seven-week old Sprague-Dowley rats were anesthetized with urethane (1 g/kg, i.p.). Catheters were introduced into the femoral artery and vein for the measurement of arterial pressure and the administration of the test compound, respectively. Blood pressure was recorded from femoral artery using a transducer coupled to the Biophysiograph 180 System (San-Ei Instrument Co., Ltd.). Inhibitory activity of the test compound was shown in the following Table as the inhibition percentage of the synthetic PAF induced hypotension. PAF was administered intravenously at a dose of 1 μg/kg. The test compound

was administered intravenously 3 minutes before PAF injection.

Test result

| Test compound No. | Dose (mg/kg) | Inhibition maximum (%) of hypotensive effect |
|---|---|---|
| 1 | 10 | 72 |

Test 3 (Inhibition of vascular permeability increase induced by intradermally injected PAF in mice)

Test method

Six-week old male ddY mice were used. The test compound was administered intraperitoneally 15 minutes before the Evans blue injection (0.125% solution in saline, 0.2 ml/mouse, i.v.).

PAF (50 ng, 50 µl per site) was injected intradermally to the depilated back of the mice 5 minutes after the Evans blue injection.

The inhibitory activity of the test compound was monitored 30 minutes after the PAF injection by assessment of blueing reaction with Evans blue dye in mice skin.

Test result

| Test compound No. | Dose (mg/kg) | Inhibition of vascular permeability increase (%) |
|---|---|---|
| 1 | 1 | 35 |

Test 4 (Inhibition of vascular permeability increase induced by intradermally injected PAF in mice)

Test method

Five-week old male ddY mice were used. The test compound suspended in 0.5% methylcellulose suspension was administered orally 30 minutes before the Evans blue injection (0.125% solution in saline, 10 ml/kg, i.v.).

PAF (50 ng, 50 µl per site) was injected intradermally to the depilated back of the mice 5 minutes after the Evans blue injection.

The inhibitory activity of the test compound was monitored 30 minutes after the PAF injection by assessment of blueing reaction with Evans blue dye in mice skin.

Test result

| Test compound No. | Dose (mg/kg) | Inhibition of vascular permeability increase (%) |
|---|---|---|
| 1 | 100 | 56.7 |

The compound (I) or pharmaceutically acceptable salt thereof in admixture with pharmaceutically acceptable carriers can be administered orally or parenterally to mammals including human being in a form of a pharmaceutical composition such as capsules, tablets, granules, powders buccal tablets, sublingual tablets, and solution.

The pharmaceutically acceptable carriers may include various organic or inorganic carrier materials, which are conventionally used for pharmaceutical purpose, such as excipient (e.g. sucrose, starch, mannit, sorbit,

lactose, glucose, cellulose, talc, calcium phosphate, calcium carbonate, etc.), binding agent (cellulose, methyl cellulose, hydroxypropylcellulose, polypropylpyrrolidone, gelatin, gum arabic, polyethyleneglycol, sucrose, starch, etc.), disintegrator (e.g. starch, carboxymethyl cellulose, calcium salt of carboxymethyl cellulose, hydroxypropyl-starch, sodium glycole-starch, sodium bicarbonate, calcium phosphate, calcium citrate, etc.), lubricant (e.g. magnesium stearate, aerosil, talc, sodium laurylsulfate, etc.), flavoring agent (e.g. citric acid, mentol, ammonium salt of glycyrrhizin, glycine, organge powders, etc.), preservative (sodium benzoate, sodium bisulfite, methylparaben, propylparaben, etc.), stabilizer (citric acid, sodium citrate, acetic acid, etc.), suspending agent (e.g. methyl cellulose, polyvinylpyrrolidone, aluminum stearate, etc.), dispersing agent [e.g. surface active agent, etc.], aqueous diluting agent (e.g. water), base wax (e.g. cacao butter, polyethyleneglycol, white petrolatum, etc.).

A dosage of the object compound is to be varied depending on various factors such as kind of diseases, weight and/or age of a patient, and further the kind of administration route.

The optimal dosage of the compound (I) is usually selected from a dose range of 1 mg - 1 g/day, preferably 10 mg - 500 mg/day.

The total daily amount mentioned above may be divisionally given to the patient at the interval of 6-12 hours per day.

The following Examples are given for the purpose of illustrating this invention.

Example 1

(1) To a solution of (αR,βR)-1,β-dimethyltryptophan methyl ester (6.36 g) and $N^{\alpha}$-t-butoxycarbonyl-3-(2-pyridyl)-D-alanine (6.65 g) in dry methylene chloride (250 ml) was added dicyclohexylcarbodiimide (5.15 g), and resulting mixture was stirred for 1.5 hours at room temperature. After evaporation of the solvent, crude residue was taken up with ethyl acetate (200 ml), filtered, washed with water and saturated sodium bicarbonate solution, dried over magnesium sulfate, treated with active charcoal, and evaporated. $N^{\alpha}$-[N-t-Butoxycarbonyl-3-(2-pyridyl)-D-alanyl]-1,β-dimethyl-D-tryptophan methyl ester (10.54 g) was obtained as an oil.

IR (Neat) : 1715, 1695, 1670 $cm^{-1}$

(2) A mixture of $N^{\alpha}$-[N-t-butoxycarbonyl-3-(2-pyridyl)-D-alanyl]-1,β-dimethyl-D-tryptophan methyl ester (10.54 g) and trifluoroacetic acid (80 ml) was stirred on an ice bath for 30 minutes. After removal of the solvent, resultant crude residue was dissolved in ethanol (150 ml) and saturated ammonia in ethanol (150 ml) was added to the solution and resultant mixture was stirred for 16 hours at ambient temperature. After evaporation of the solvent, chloroform (200 ml) was added to the mixture. The insoluble material was filtered off and again solvent was evaporated in vacuo to give an oil which was chromatographed on silica gel (250 g, chloroform-methanol 20:1 as eluent) to afford (3R,6R)-3-[(R)-1-(1-methylindol-3-yl)ethyl]-6-(2-pyridylmethyl)piperazine-2,5-dione.
Crystallization from ethanol yielded 3.14 g of the crystals.

mp : 227-228°C

IR (Nujol) : 3200, 1675, 1655 $cm^{-1}$

Example 2

The following compounds were prepared in a similar manner to that of Example 1.

(1) (3R,6RS)-3-(1-Methylindol-3-yl)methyl-6-(3-pyridylmethyl)piperazine-2,5-dione
mp : 231-238°C
IR (Nujol) : 3200, 1670 $cm^{-1}$

(2) (3R,6R)-3-(1-Methylindol-3-yl)methyl-6-(4-pyridylmethyl)piperazine-2,5-dione
mp : 263-271°C

(3) (3R,6R)-3-Butyl-6-(1-naphthylmethyl)piperazine-2,5-dione
mp : 249-250°C
IR (Nujol) : 3190, 3040, 1670, 1660 $cm^{-1}$

(4) (3R,6R)-3-[(R)-1-(1-Ethylindol-3-yl)ethyl]-6-(2-pyridylmethyl)piperazine-2,5-dione
mp : 237-240°C
IR (Nujol) : 3200, 1675, 1660 $cm^{-1}$

(5) (3R,6R)-3-[(R)-1-(5-Chloro-1-methylindol-3-yl)-ethyl]-6-(2-pyridylmethyl)piperazine-2,5-dione
mp : 229-234°C
IR (Nujol) : 3210, 1680, 1660 $cm^{-1}$

(6) (3R,6R)-3-[(R)-1-(5-Methoxy-1-methylindol-3-yl)-ethyl]-6-(2-pyridylmethyl)piperazine-2,5-dione
mp : 220-222°C
IR ($CHCl_3$) : 3400, 1675 $cm^{-1}$

(7) (3R,6R)-3-[(R)-1-(1-Methylindol-3-yl)ethyl]-6-(1,3-thiazol-4-ylmethyl)piperazine-2,5-dione
mp : 238-241°C
IR ($CHCl_3$) : 3400, 1680 $cm^{-1}$

Example 3

(3R,6R)-3-[(R)-1-(1-Methylindol-3-yl)ethyl]-6-(2-pyridylmethyl)piperazine-2,5-dione (0.20 g) was dissolved in a mixture of ethanol (5 ml), chloroform (2 ml), and 1.4N ethanolic hydrogen chloride (0.5 ml) and solvent was evaporated in vacuo. The resultant crude residue was recrystallized from acetone to give hydrochloric acid salt of (3R,6R)-3-[(R)-1-(1-methylindol-3-yl)ethyl]-6-(2-pyridylmethyl)piperazine-2,5-dione (0.12 g).

mp : 180°C

Example 4

A mixture of (3R,6R)-3-[(R)-1-(1-methylindol-3-yl)ethyl]-6-(2-pyridylmethyl)piperazine-2,5-dione (0.10 g) and methyl iodide (5 ml) was stirred under reflux for 4 days. After evaporation of the excess methyl iodide, crude oily residue was washed with chloroform and ether, and dried in vacuo to give (3R,6R)-3-[(R)-1-(1-methylindol-3-yl)ethyl]-6-(1-methyl-2-pyridiniomethyl)piperazine-2,5-dione iodide as a solid (0.12 g).

NMR (DMSO-$d_6$, δ) : 1.20 (1H, m), 1.45 (3H, d, J=7Hz), 2.55 (1H, m), 3.70 (1H, m), 3.78 (3H, s), 3.81 (3H, s), 4.05 (2H, m), 5.95 (1H, d, J=8.5Hz), 7.15 (3H, m), 7.40 (1H, d, J=8Hz), 7.65 (1H, d, J=8Hz), 7.73 (1H, d, J=1Hz), 7.85 (1H, m), 8.13 (1H, m), 8.32 (1H, s), 8.80 (2H, m)

Example 5

To a mixture of (3R,6R)-3-[(R)-1-(1-methylindol-3-yl)ethyl]-6-(1-methyl-2-pyridiniomethyl)piperazine-2,5-dione iodide (0.12 g), methanol (1.2 ml), and water (1.2 ml) was added sodium borohydride (0.12 g).

The mixture was stirred on an ice bath for 1 hour, diluted with water (10 ml), and set aside in a refrigerator overnight. A precipitated solid was filtered, washed with water, and dried in vacuo to afford (3R,6R)-3-[(R)-1-methylindol-3-yl)ethyl]-6-[(RS)-1-methyl-1,2,3,6-tetrahydropyridin-6-yl-methyl]piperazine-2,5-dione (0.03 g).

mp : 235-238°C

IR (Nujol) : 3200, 1670, 1660 $cm^{-1}$

Example 6

(1) To a solution of 5-chloro-1-ethylindole (6.17 g) and methyl (2R,3R)-N-benzyloxycarbonyl-3-methyl-2-aziridinecarboxylate (9.87 g) in dry methylene chloride (100 ml) which was cooled on an ice bath, was added boron trifluoride etherate (4 ml). The mixture was stirred for five minutes and washed with saturated sodium bicarbonate solution, treated with active charcoal, and dried over magnesium sulfate. After removal of the solvent, the crude residue was chromatographed on silica gel (100 g) (toluene as eluent) to give (αR,βR)-$N^{\alpha}$-benzyloxycarbonyl-5-chloro-1-ethyl-β-methyltryptophan methyl ester (4.17 g) as an oil.

$[\alpha]_{20}^{D}$ -31.1° (in MeOH, C=1.3)

(2) A solution of (αR,βR)-$N^{\alpha}$-Benzyloxycarbonyl-5-chloro-1-ethyl-β-methyltryptophan methyl ester (4.17 g) in methanol (100 ml) containing 10% paradium on charcoal (0.5 g) was hydrogenated. After removal of the catalyst and evaporation of the solvent, (αR,βR)-5-chloro-1-ethyl-β-methyltryptophan methyl ester (2.45 g) was obtained as a yellow amorphous powder.

$[\alpha]_{20}^{D}$ -37.1° (in MeOH, C=1.5)

(3) To a solution of (αR,βR)-5-chloro-1-ethyl-β-methyltryptophan methyl ester (1.24 g) and N-t-butoxycarbonyl-3-(2-pyridyl)-D-alanine (1.12 g) in dry methylene chloride (30 ml) which was cooled on an ice bath, was added a solution of N,N'-dicyclohexylcarbodiimide (0.9 g) in dry methylene chloride (5 ml). The mixture was stirred overnight at room temperature. After removal of the solvent, the crude residue was taken up with a mixture of ethyl acetate and water, filtered, and the organic layer was washed with saturated sodium bicarbonate solution, dried over magnesium sulfate, and solvent was evaporated off to give $N^{\alpha}$-[N-t-butoxycarbonyl-3-(2-pyridyl)-D-alanyl]-(αR,βR)-5-chloro-1-ethyl-β-methyltryptophan methyl ester (2.29 g). To this compound was added trifluoroacetic acid (10 ml) and the mixture was stirred for 30 minutes on an ice bath. After evaporation of the solvent, the crude residue was dissolved in chloroform (50 ml), washed with saturated sodium bicarbonate solution, treated with active charcoal, dried over magnesium sulfate, and the solvent was evaporated off to give an oil (1.43 g). The crude material was dissolved in ethanol (10 ml) and saturated alcoholic ammonia (10 ml) was added. The resulting mixture was allowed to stand for 3 days at room temperature and concentrated in vacuo. The crude residue was chromatographed on silica gel (70 g) (chloroform and 50:1 mixture of chloroform and methanol as eluent) to give an oil (0.56 g) which was crystallized from ethanol to afford (3R,6R)-3-[(R)-1-(5-chloro-1-ethylindol-3-yl)ethyl]-6-(2-pyridylmethyl)piperazine-2,5-dione (0.34 g).

mp : 223-226°C

IR (Nujol) : 3210, 1675, 1655 $cm^{-1}$

Example 7

(1) The following compound was prepared in a similar manner to that of Example 6(1).

(αR,βR)-$N^{\alpha}$-Benzyloxycarbonyl-β-methyl-1-propyltryptophan methyl ether

$[\alpha]^{D}_{20}$ -22.4° (in MeOH, C=1.8)

(2) The following compound was prepared in a similar manner to that of Example 6(2).

(αR,βR)-β-Methyl-1-propyltryptophan methyl ester

$[\alpha]^{D}_{20}$ -26.4° (in MeOH, C=1.1)

(3) The following compound was prepared in a similar manner to that of Example 6(3).

(3R,6R)-3-[(R)-1-(1-Propylindol-3-yl)ethyl]-6-(2-pyridylmethyl)piperazine-2,5-dione

mp : 178-181°C

IR (Nujol) : 1650 $cm^{-1}$

(4) The following compound was prepared in a similar manner to that of Example 6(3).

(3R,6S)-3-[(R)-1-(1-propylindol-3-yl)ethyl]-6-(2-pyridylmethyl)piperazine-2,5-dione

mp : 175-180°C

IR (Nujol) : 1670 $cm^{-1}$

Example 8

(1) The following compound was prepared in a similar manner to that of Example 6(1).

(αR,βR)-$N^{\alpha}$-Benzyloxycarbonyl-1,β-dimethyl-5-fluorotryptophan methyl ester

$[\alpha]^{D}_{20}$ 32.5° (in MeOH, C=1.2)

(2) The following compound was prepared in a similar manner to that of Example 6(2).

(αR,βR)-1,β-Dimethyl-5-fluorotryptophan methyl ester

$[\alpha]^{D}_{20}$ -22.6° (in MeOH, C=0.4)

(3) The following compound was prepared in a similar manner to that of Example 6(3).

(3R,6R)-3-[(R)-1-(5-Fluoro-1-methylindol-3-yl)-ethyl]-6-(2-pyridylmethyl)piperazine-2,5-dione

mp : 242-248°C

IR (Nujol) : 1665 $cm^{-1}$

Example 9

The following compounds were prepared in a similar manner to that of Example 6(3).

(1) (3R,6S)-3-[(R)-1-(5-Fluoro-1-methylindol-3-yl)-ethyl]-6-(2-pyridylmethyl)piperazine-2,5-dione

mp : 243-248°C

IR (Nujol) : 1670 $cm^{-1}$

(2) (3R,6S)-3-[(R)-1-(5-Chloro-1-ethylindol-3-yl)-ethyl]-6-(2-pyridylmethyl)piperazine-2,5-dione

mp : 192-194°C

IR (Nujol) : 1670 $cm^{-1}$

(3) (3R,6R)-3-[(R)-1-(5-Chloro-1-ethylindol-3-yl)ethyl]-6-(4-thiazolylmethyl)piperazine-2,5-dione

mp : 190-193°C

IR (Nujol) : 1670 $cm^{-1}$

(4) (3R,6S)-3-[(R)-1-(5-Chloro-1-ethylindol-3-yl)-ethyl]-6-(4-thiazolylmethyl)piperazine-2,5-dione
mp : 218-220°C
IR (Nujol) : 1675 $cm^{-1}$

Example 10

(1) The following compound was prepared in a similar manner to that of Example 6(1).

(αR,βR)-$N^{\alpha}$-Benzyloxycarbonyl-1-butyl-5-fluoro-β-methyltryptophan methyl ester
$[\alpha]^{D}_{20}$ -7.16° (in MeOH, C=1.65)

(2) The following compound was prepared in a similar manner to that of Example 6(2).

(αR,βR)-1-Butyl-5-fluoro-β-methyltryptophan methyl ester
$[\alpha]^{D}_{20}$ -7.99° (in MeOH, C=1.40)

(3) The following compound was prepared in a similar manner to that of Example 6(3).

(3R,6R)-3-[(R)-1-(1-Butyl-5-fluoroindol-3-yl)-ethyl]-6-(2-pyridylmethyl)piperazine-2,5-dione
mp : 155-160°C
IR (Nujol) : 1680 $cm^{-1}$

Example 11

The following compounds were prepared in a similar manner to that of Example 6(3).

(1) (3R,6S)-3-[(R)-1-(1-Butyl-5-fluoroindol-3-yl)-ethyl]-6-(2-pyridylmethyl)piperazine-2,5-dione
mp : 173-176°C
IR (Nujol) : 1665 $cm^{-1}$

(2) (3R,6R)-3-[(R)-1-(1-Butyl-5-fluoroindol-3-yl)-ethyl]-6-(4-thiazolylmethyl)piperazine-2,5-dione
mp : 180-183°C
IR (Nujol) : 1670 $cm^{-1}$

Example 12

(1) The following compound was prepared in a similar manner to that of Example 6(1).

(αR,βR)-$N^{\alpha}$-Benzyloxycarbonyl-1-butyl-5-chloro-2,β-dimethyltryptophan methyl ester
$[\alpha]_{20}^{D}$ 2.72° (in MeOH, C=1.65)

(2) The following compound was prepared in a similar manner to that of Example 6(2).

(αR,βR)-5-Chloro-2,β-dimethyl-1-butyltryptophan methyl ester
$[\alpha]_{20}^{D}$ -1.06° (in MeOH, C=1.45)

(3) The following compound was prepared in a similar manner to that of Example 6(3).

(3R,6R)-3-[(R)-1-(1-Butyl-5-chloro-2-methylindol-3-yl)ethyl]-6-(2-pyridylmethyl)piperazine-2,5-dione
mp : 155-168°C
IR (Nujol) : 1680 $cm^{-1}$

Example 13

The following compounds were prepared in a similar manner to that of Example 6(3).

(1) (3R,6S)-3-[(R)-1-(1-Butyl-5-chloro-2-methylindol-3-yl)ethyl]-6-(2-pyridylmethyl)piperazine-2,5-dione
mp : 183-190°C
IR (Nujol) : 1660 $cm^{-1}$

(2) (3R,6R)-3-[(R)-1-(1-Butyl-5-chloro-2-methylindol-3-yl)ethyl]-6-(4-thiazolylmethyl)piperazine-2,5-dione
mp : 105-110°C
IR (Nujol) : 1670 $cm^{-1}$

Example 14

(1) The following compound was prepared in a similar to that of Example 6(1).

($\alpha$R,$\beta$R)-$N^{\alpha}$-Benzyloxycarbonyl-5,$\beta$-dimethyl-1-propyltryptophan methyl ester
$[\alpha]_{20}^{D}$ -43.30° (in MeOH, C=0.86)

(2) The following compound was prepared in a similar manner to that of Example 6(2).

($\alpha$R,$\beta$R)-5,$\beta$-Dimethyl-1-propyltryptophan methyl ester
$[\alpha]_{20}^{D}$ -38.34° (in MeOH, C=1.15)

(3) The following compound was prepared in a similar manner to that of Example 6(3).

(3R,6R)-3-[(R)-1-(5-Methyl-1-propylindol-3-yl)-ethyl]-6-(2-pyridylmethyl)piperazine-2,5-dione
mp : 214-215°C
IR (Nujol) : 3200, 1665 $cm^{-1}$

Example 15

The following compounds were prepared in a similar manner to that of Example 6(3).

(1) (3R,6S)-3-[(R)-1-(5-Methyl-1-propylindol-3-yl)ethyl]-6-(2-pyridylmethyl)piperazine-2,5-dione
mp : 192-193°C
IR (Nujol) : 3200, 1670 $cm^{-1}$.

(2) (3R,6R)-3-[(R)-1-(5-Methyl-1-propylindol-3-yl)-ethyl]-6-(4-thiazolylmethyl)piperazine-2,5-dione
mp : 201.5-202.5°C
IR (Nujol) : 3200, 1665 $cm^{-1}$

Example 16

(1) The following compound was prepared in a similar manner to that of Example 6(1).

(αR,βR)-$N^{\alpha}$-Benzyloxycarbonyl-1-propyl-2,5,β-trimethyltryptophan methyl ester
$[\alpha]^{D}_{20}$ -1.45° (in MeOH, C=0.9)

(2) The following compound was prepared in a similar manner to that of Example 6(2).

(αR,βR)-1-Propyl-2,5,β-trimethyltryptophan methyl ester
$[\alpha]^{D}_{20}$ -0.71° (C=0.556 in MeOH)

(3) The following compound was prepared in a similar manner to that of Example 6(3).

(3R,6R)-3-[(R)-1-(2,5-Dimethyl-1-propylindol-3-yl)ethyl]-6-(2-pyridylmethyl)piperazine-2,5-dione
mp : 231.5-232.0°C
IR (Nujol) : 3340, 3220, 3100, 1680, 1650 $cm^{-1}$

Example 17

The following compounds were prepared in a similar manner to that of Example 6(3).

(3R,6S)-3-[(R)-1-(2,5-Dimethyl-1-propylindol-3-yl)ethyl]-6-(2-pyridylmethyl)piperazine-2,5-dione
mp : 218.0°C
IR (Nujol) : 3200, 1665 $cm^{-1}$

Example 18

(1) The following compound was prepared in a similar manner to that of Example 6(1).

($\alpha$R,$\beta$R)-N$^{\alpha}$-Benzyloxycarbonyl-1,2,$\beta$-trimethyltryptophan methyl ester

$[\alpha]^{D}_{20}$ -0.70° (in MeOH, C=1.7)

(2) The following compound was prepared in a similar manner to that of Example 6(2).

($\alpha$R,$\beta$R)-1,2,$\beta$-Trimethyltryptophan methyl ester

$[\alpha]^{D}_{20}$ -18.4° (in MeOH, C=1.0)

(3) The following compound was prepared in a similar manner to that of Example 6(3).

(3R,6R)-3-[(R)-1-(1,2-Dimethylindol-3-yl)ethyl]-6-(2-pyridylmethyl)piperazine-2,5-dione

mp : 265-269°C

IR (Nujol) : 1675, 1650 $cm^{-1}$

Example 19

The following compound was prepared in a similar manner to that of Example 6(3).

(3R,6S)-3-[(R)-1-(1,2-Dimethylindol-3-yl)ethyl]-6-(2-pyridylmethyl)piperazine-2,5-dione

mp : 216-219°C

IR (Nujol) : 1675 $cm^{-1}$

Example 20

(1) The following compound was prepared in a similar manner to that of Example 6(1).

(αR,βR)-$N^{\alpha}$-Benzyloxycarbonyl-1,5,β-trimethyltryptophan methyl ether

$[\alpha]_{20}^{D}$ -19.2° (in MeOH, C=1.2)

(2) The following compound was prepared in a similar manner to that of Example 6(2).

(αR,βR)-1,5,β-Trimethyltryptophan methyl ester

$[\alpha]_{20}^{D}$ -45.3° (in MeOH, C=1.2)

(3) The following compound was prepared in a similar manner to that of Example 6(3).

(3R,6R)-3-[(R)-1-(1,5-Dimethylindol-3-yl)ethyl]-6-(2-pyridylmethyl)piperazine-2,5-dione

mp : 256-257°C

IR (Nujol) : 3200, 1650 $cm^{-1}$

Example 21

The following compound was prepared in a similar manner to that of Example 6(3).

(3R,6S)-3-[(R)-1-(1,5-Dimethylindol-3-yl)ethyl]-6-(2-pyridylmethyl)piperazine-2,5-dione

mp : 260-262°C

IR (Nujol) : 3200, 1660 $cm^{-1}$

Example 22

(1) The following compound was prepared in a similar manner to that of Example 6(1).

(αR,βR)-$N^{\alpha}$-Benzyloxycarbonyl-5-chloro-2,β-dimethyl-1-propyltryptophan methyl ester

$[\alpha]_{20}^{D}$ 17.98° (in MeOH, C=1.3)

(2) The following compound was prepared in a similar manner to that of Example 6(2).

(αR,βR)-5-Chloro-2,β-dimethyl-1-propyltryptophan methyl ester

$[\alpha]_{20}^{D}$ -0.50° (in MeOH, C=1.0)

(3) The following compound was prepared in a similar manner to that of Example 6(3).

(3R,6R)-3-[(R)-1-(5-Chloro-2-methyl-1-propylindol-3-yl)ethyl]-6-(2-pyridylmethyl)piperazine-2,5-dione

mp : 195°C

IR (Nujol) : 1660 $cm^{-1}$

Example 23

The following compounds were prepared in a similar manner to that of Example 6(3).

(1) (3R,6S)-3-[(R)-1-(5-Chloro-2-methyl-1-propylindol-3-yl)ethyl]-6-(2-pyridylmethyl)piperazine-2,5-dione

mp : 197-198°C

IR (Nujol) : 1670 $cm^{-1}$

(2) (3R,6R)-3-[(R)-1-(5-Chloro-2-methyl-1-propylindol-3-yl)ethyl]-6-(4-thiazolylmethyl)piperazine-2,5-dione

mp : 160°C

IR (Nujol) : 1660 $cm^{-1}$

Example 24

(1) The following compound was prepared in a similar manner to that of Example 6(1).

(αR,βR)-$N^{\alpha}$-Benzyloxycarbonyl-1-butyl-2,β-dimethyltryptophan methyl ester

$[\alpha]^{D}_{20}$ -3.27° (in MeOH, C=1.365)

(2) The following compound was prepared in a similar manner to that of Example 6(2).

(αR,βR)-1-Butyl-2,β-dimethyltryptophan methyl ester

$[\alpha]^{D}_{20}$ -7.21° (in MeOH, C=1.065)

(3) The following compound was prepared in a similar manner to that of Example 6(3).

(3R,6R)-3-[(R)-1-(1-Butyl-2-methylindol-3-yl)-ethyl]-6-(4-thiazolylmethyl)piperazine-2,5-dione

mp : 200-201°C

IR ($CHCl_3$) : 1680 $cm^{-1}$

Example 25

The following compounds were prepared in a similar manner to that of Example 6(3).

(1) (3R,6R)-3-[(R)-1-(1-Butyl-2-methylindol-3-yl)-ethyl]-6-(2-pyridylmethyl)piperazine-2,5-dione

mp : 210-210.5°C

IR ($CHCl_3$) : 1677 $cm^{-1}$

(2) (3R,6S)-3-[(R)-1-(1-Butyl-2-methylindol-3-yl)ethyl]-6-(2-pyridylmethyl)piperazine-2,5-dione

mp : 150°C

IR ($CHCl_3$) : 1680 $cm^{-1}$

Example 26

(1) The following compound was prepared in a similar manner to that of Example 6(1).

(αR,βR)-$N^{\alpha}$-Benzyloxycarbonyl-β-methyl-1-isobutyl-tryptophan methyl ester

$[\alpha]_{20}^{D}$ -33.14 (in MeOH, C=1.095)

(2) The following compound was prepared in a similar manner to that of Example 6(2).

(αR,βR)-β-Methyl-1-isobutyltryptophan methyl ester

$[\alpha]_{20}^{D}$ -33.19 (in MeOH, C=1.03)

(3) The following compound was prepared in a similar manner to that of Example 6(3).

(3R,6R)-3-[(R)-1-(1-Isobutylindol-3-yl)ethyl]-6-(2-pyridylmethyl)piperazine-2,5-dione

mp : 204°C

IR ($CHCl_3$) : 1678 $cm^{-1}$

Example 27

The following compounds were prepared in a similar manner to that of Example 6(3).

(1) (3R,6S)-3-[(R)-1-(1-Isobutylindol-3-yl)ethyl]-6-(2-pyridylmethyl)piperazine-2,5-dione

mp : 232°C

IR ($CHCl_3$) : 1680 $cm^{-1}$

(2) (3R,6R)-3-[(R)-1-(1-Isobutylindol-3-yl)ethyl]-6-(4-thiazolylmethyl)piperazine-2,5-dione

mp : 126-127.5°C

IR ($CHCl_3$) : 1679 $cm^{-1}$

Example 28

(1) To a solution of (αR,βR)-$N^{\alpha}$-benzyloxycarbonyl-

1,β-dimethyltryptphan methyl ester (6.0 g) in methanol (60 ml) was added a solution of sodium hydroxide (1.26 g) in water (10 ml), and the resulting mixture was stirred at room temperature for 8 hours. The reaction mixture was acidified with 10% hydrochloric acid, concentrated to a volume of 30 ml, extracted with ethyl acetate (30 ml x 2). Combined organic extracts were washed with water, dried over magnesium sulfate, treated with active charcoal, and evaporated in vacuo to give (αR,βR)-$N^{\alpha}$-benzyloxycarbonyl-1,β-dimethyltryptophan (5.29 g) as an oil.

$[\alpha]^{D}_{20}$ -32.4° (in MeOH, C=1.2)

(2) A mixture of (αR,βR)-$N^{\alpha}$-benzyloxycarbonyl-1,β-dimethyltryptophan (1.95 g), N,N'-disuccinimidyl carbonate (1.5 g), and pyridine (0.46 g) in dry acetonitrile (15 ml) was stirred at room temperature for 18 hours. After evaporation of the solvent, the residue was dissolved in ethyl acetate (30 ml), washed with water (10 ml), 10% hydrochloric acid (10 ml), water (5 ml), saturated sodium bicarbonate solution (10 ml), and saturated sodium chloride solution (5 ml), dried over magnesium sulfate, and evaporated in vacuo to give an oil (3.38 g). The residual oil (3.38 g) and 3-(2-pyridyl)-D-alanine ethyl ester dihydrochloride (1.56 g) were dissolved in a mixture of dioxane (30 ml) and water (15 ml). The mixture was neutralized with triethylamine (1.78 g) and stirred at room temperature overnight. After evaporation of the solvent, the reaction mixture was extracted with ethyl acetate (30 ml x 2). The combined organic extract was washed with water, saturated sodium bicarbonate solution, and saturated sodium chloride solution dried over magnesium sulfate, and evaporated in vacuo to give $N^{\alpha}$-[(αR,βR)-N-

benzyloxycarbonyl-1,β-dimethyltryptophyl]-3-(2-pyridyl)-D-alanine ethyl ester (2.78 g). The resultant compound in methanol (28 ml) was hydrogenated over 10% paradium-charcoal (0.28 g) to give an oil (2.05 g), which was dissolved in ethanol (10 ml) and saturated ammonia-ethanol solution (10 ml) was added. The mixture was allowed to stand at room temperature for 18 hours and solvent was evaporated in vacuo. Crude residue was washed with ethyl acetate to give crystals (1.40 g). The crystal was recrystallized from ethanol to give (3R,6R)-3-[(R)-1-(1-methylindol-3-yl)ethyl]-6-(2-pyridylmethyl)piperazine-2,5-dione (1.24 g).

Physicochemical properties of this compound were identical with that of the object compound of Example 1.

Example 29

The following compounds were prepared in a similar manner to that of Example 28(2).

(1) (3R,6R)-3-[(R)-1-(1-Methylindol-3-yl)ethyl]-6-(2-quinolylmethyl)piperazine-2,5-dione
mp : 261-267°C
IR (Nujol) : 3190, 1670, 1655 $cm^{-1}$

(2) (3R,6S)-3-[(R)-1-(1-Methylindol-3-yl)ethyl]-6-(2-quinolylmethyl)piperazine-2,5-dione
mp : 211-218°C
IR (Nujol) : 3190, 1670 $cm^{-1}$

Example 30

(1) The following compound was prepared in a similar manner to that of Example 6(1).

(αR,βR)-$N^{\alpha}$-Benzyloxycarbonyl-1,β-dimethyl-2-phenyltryptophan methyl ester

$[\alpha]_{20}^{D}$ -0.86° (in MeOH, C=1.5)

(2) The following compound was prepared in a similar manner to that of Example 6(2).

(αR,βR)-1,β-Dimethyl-2-phenyltryptophan methyl ester

$[\alpha]_{20}^{D}$ 18.1° (in MeOH, C=1.1)

(3) The following compounds were prepared in a similar manner to that of Example 6(3).

(a) (3R,6R)-3-[(R)-1-(1-Methyl-2-phenylindol-3-yl)-ethyl]-6-(2-pyridylmethyl)piperazine-2,5-dione

mp : 233-236°C

IR (Nujol) : 1680 $cm^{-1}$

(b) (3R,6S)-3-[(R)-1-(1-Methyl-2-phenylindol-3-yl)-ethyl]-6-(2-pyridylmethyl)piperazine-2,5-dione

mp : 222-223°C

IR (Nujol) : 1675 $cm^{-1}$

Example 31

(3R,6R)-3-[(R)-1-(1-Methylindol-3-yl)ethyl]-6-(2-pyridylmethyl)piperazine-2,5-dione (5.0 g) was dissolved in water (10 ml) containing sulfuric acid

(1.40 g). After evaporation of the water, crude residue was recrystallized from ethanol to give sulfuric acid salt of (3R,6R)-3-[(R)-1-(1-methylindol -3-yl)-ethyl]-6-(2-pyridylmethyl)piperazine-2,5-dione (3.32 g).

mp : 139°C

Example 32

(3R,6R)-3-[(R)-1-(1-Methylindole-3-yl)ethyl]-6-(2-pyridylmethyl)piperazine-2,5-dione (5 g) was dissolved in water (10 ml) containing oxalic acid (1.91 g). After evaporation of the water, crude solid was recrystallized from ethanol to give oxalic acid salt of (3R,6R)-3-[(R)-1-(1-methylindol -3-yl)ethyl]-6-(2-pyridylmethyl)piperazine-2,5-dione (5.67 g).

mp : 170-173°C.

Example 33

(1) The following compound was prepared in a similar manner to that of Example 28(1).

($\alpha$R,$\beta$R)-$N^{\alpha}$-t-Butoxycarbonyl-5-bromo-1-ethyl-$\beta$-methyltryptophan

$[\alpha]_{20}^{D}$ -41.2° (in MeOH, c=1.2)

(2) The following compound was prepared in a similar manner to that of Example 28(2).

(3R,6R)-3-[(R)-1-(5-Bromo-1-ethylindol-3-yl)ethyl]-6-(2-pyridylmethyl)piperazine-2,5-dione

mp: 232-233°C

IR(Nujol): 1645 $cm^{-1}$

Example 34

(1) The following compound was prepared in a similar manner to that of Example 28(1).

($\alpha$R,$\beta$R)-$N^{\alpha}$-t-Butoxycarbonyl-1-ethyl-5-methoxy-$\beta$-methyltryptophan

$[\alpha]_{20}^{D}$ -29.3° (in MeOH, c=1.1)

(2) The following compound was prepared in a similar manner to that of Example 28(2).
(3R,6R)-3-[(R)-1-(1-Ethyl-5-methoxyindol-3-yl)ethyl]-6-(2-pyridylmethyl)piperazine-2,5-dione
mp: 201-203°C
IR(Nujol): 1650cm$^{-1}$

Example 35

(1) The following compound was prepared in a similar manner to that of Example 28(1).
(αR,βR)-N$^{\alpha}$-t-Butoxycarbonyl-1-methyl-β-phenyltryptophan
IR($CHCl_3$): 1720cm$^{-1}$

(2) The following compound was prepared in a similar manner to that of Example 28(2).
(3R,6R)-3-[(R)-α-(1-Methylindol-3-yl)benzyl]-6-(2-pyridylmethyl)piperazine-2,5-dione
mp: 238-240°C

Example 36

The following compound was prepared in a similar manner to that of Example 28(2).
(3R,6S)-3-[(R)-α-(1-Methylindol-3-yl)benzyl]-6-(2-pyridylmethyl)piperazine-2,5-dione
mp: 265-268°C

Example 37

(1) The following compound was prepared in a similar manner to that of Example 28(1).
(αR,βR)-N$^{\alpha}$-t-Butoxycarbonyl-5-benzyloxy-1-ethyl-β-

methyltryptophan

$[\alpha]^{D}_{20}$ -52.4° (in MeOH, c=1.8)

(2) The following compound was prepared in a similar manner to that of Example28(2).

(3R,6R)-3-[(R)-1-(5-Benzyloxy-1-ethylindol-3-yl)ethyl]-6-(2-pyridylmethyl)piperazine-2,5-dione

mp: 243-246°C

IR(Nujol): 1660, 1640 $cm^{-1}$

Example 38

The following compound was prepared in a similar manner to that of Example 28(2).

(3R,6S)-3-[(R)-1-(5-Benzyloxy-1-ethylindol-3-yl)ethyl]-6-(2-pyridylmethyl)piperazine-2,5-dione

mp: 195-196°C

IR(Nujol): 1670, 1650 $cm^{-1}$

Example 39

(1) The following compound was prepared in a similar manner to that of Example 28(1).

(αR,βR)-$N^{\alpha}$-t-Butoxycarbonyl-β-ethyl-1-methyltryptophan

IR(neat): 1720 $cm^{-1}$

(2) The following compound was prepared in a similar manner to that of Example 28(2).

(3R,6R)-3-[(R)-1-(1-Methylindol-3-yl)propyl]-6-(2-pyridylmethyl)piperazine-2,5-dione

mp: 217-219°C

IR(Nujol): 1680 $cm^{-1}$

Example 40

(1) The following compound was prepared in a similar manner to that of Example 28(1).

(αR,βR)-$N^{\alpha}$-Benzyloxycarbonyl-6,β-dimethyl-1-ethyl-tryptophan

$[\alpha]^{D}_{20}$ -30.3° (in MeOH, c=1.3)

IR(neat): 1700 $cm^{-1}$

(2) The following compound was prepared in a similar manner to that of Example 28(2).

(3R,6R)-3-[(R)-1-(1-Ethyl-6-methylindol-3-yl)ethyl]-6-(2-pyridylmethyl)piperazine-2,5-dione

mp: 216-217°C

IR(Nujol): 1660, 1650 $cm^{-1}$

Example 41

The following compound was prepared in a similar manner to that of Example 28(2).

(3R,6R)-3-[(R)-1-(1-Ethyl-6-methylindol-3-yl)ethyl]-6-(4-thiazolylmethyl)piperazine-2,5-dione

mp: 229-230°C

Example 42

(1) The following compound was prepared in a similar manner to that of Example 28(1).

(αR,βR)-$N^{\alpha}$-Benzyloxycarbonyl-5,β-dimethyl-1-ethyl-tryptophan

$[\alpha]^{D}_{20}$ -34.7° (in MeOH, c=1.7)

(2) The following compound was prepared in a similar manner to that of Example 28(2).

(3R,6R)-3-[(R)-1-(1-Ethyl-5-methylindol-3-yl)ethyl]-6-(2-pyridylmethyl)piperazine-2,5-dione

mp: 212°C

IR(Nujol): 1650 $cm^{-1}$

(3) The following compound was prepared in a similar manner to that of Example 32.
Hydrochloric acid salt of (3R,6R)-3-[(R)-1-(1-Ethyl-5-methylindol-3-yl)ethyl]-6-(2-pyridylmethyl)piperazine-2,5-dione
mp: 135°C
IR(Nujol): 1670, 1640 $cm^{-1}$

Example 43

(1) To a stirred suspension of sodium hydride (60%,2.10g) in dry tetrahydrofuran (30 ml) was added a solution of (αR,βR)-$N^{\alpha}$-t-butoxycarbonyl-5-chloro-1-ethyl-β-methyltryptophan (5.0 g) in dry tetrahydrofuran (20 ml) under nitrogen atomosphere on an ice bath. The resulting mixture was stirred at room temperature for 1 hour and methyl iodide (3.28 ml) was added to the mixture. After additional stirring overnight, the reaction mixture was poured into water, washed with ether, acidified with 1N hydrochloric acid, and extracted with ethyl acetate to give (αR,βR)-$N^{\alpha}$-t-butoxycarbonyl-5-chloro-1-ethyl-$N^{\alpha}$,β-dimethyltryptophan as an oil (4.8 g)
IR(neat): 1720 $cm^{-1}$

(2) The following compound was prepared in a similar manner to that of Example 28(2).
(3R,6R)-3-[(R)-1-(5-Chloro-1-ethylindol-3-yl)ethyl]-4-methyl-6-(2-pyridylmethyl)piperazine-2,5-dione
mp: 173-178°C
IR(Nujol): 1660, 1680 $cm^{-1}$

Example 44

The following compounds were prepared in a similar manner to that of Example 6(3).

(1) (3R,6R)-3-[(R)-1-(1-Methylindol-3-yl)ethyl]-6-(2-quinolylmethyl)piperazine-2,5-dione
IR (Nujol) : 3190, 1670, 1655 $cm^{-1}$

(2) (3R,6S)-3-[(R)-1-(1-Methylindol-3-yl)ethyl]-6-(2-quinolylmethyl)piperazine-2,5-dione
IR (Nujol) : 3190, 1670 $cm^{-1}$

(3) (3R,6R)-3-[(R)-1-(5-Bromo-1-ethylindol-3-yl)ethyl]-6-(2-pyridylmethyl)piperazine-2,5-dione
IR (Nujol) : 1645 $cm^{-1}$

(4) (3R,6R)-3-[(R)-1-(1-Ethyl-5-methoxyindol-3-yl)ethyl]-6-(2-pyridylmethyl)piperazine-2,5-dione
IR (Nujol) : 1650 $cm^{-1}$

(5) (3R,6R)-3-[(R)-α-(1-Methylindol-3-yl)benzyl]-6-(2-pyridylmethyl)piperazine-2,5-dione
mp : 238-240°C

(6) (3R,6S)-3-[(R)-α-(1-Methylindol-3-yl)benzyl]-6-(2-pyridylmethyl)piperazine-2,5-dione
mp : 265-268°C

(7) (3R,6R)-3-[(R)-1-(5-Benzyloxy-1-ethylindol-3-yl)-ethyl]-6-(2-pyridylmethyl)piperazine-2,5-dione
IR (Nujol) : 1660, 1640 $cm^{-1}$

(8) (3R,6S)-3-[(R)-1-(5-Benzyloxy-1-ethylindol-3-yl)-ethyl]-6-(2-pyridylmethyl)piperazine-2,5-dione
IR (Nujol) : 1670, 1650 $cm^{-1}$

(9) (3R,6R)-3-[(R)-1-(1-Methylindol-3-yl)propyl]-6-(2-pyridylmethyl)piperazine-2,5-dione
IR (Nujol) : 1680 $cm^{-1}$

(10) (3R,6R)-3-[(R)-1-(1-Ethyl-6-methylindol-3-yl)ethyl]-6-(2-pyridylmethyl)piperazine-2,5-dione
IR (Nujol) : 1660, 1650 $cm^{-1}$

(11) (3R,6R)-3-[(R)-1-(1-Ethyl-6-methylindol-3-yl)ethyl]-6-(4-thiazolylmethyl)piperazine-2,5-dione
mp : 229-230°C

(12) (3R,6R)-3-[(R)-1-(1-Ethyl-5-methylindol-3-yl)ethyl]-6-(2-pyridylmethyl)piperazine-2,5-dione
IR (Nujol) : 1650 $cm^{-1}$

(13) (3R,6R)-3-[(R)-1-(5-Chloro-1-ethylindol-3-yl)ethyl]-4-methyl-6-(2-pyridylmethyl)piperazine-2,5-dione
IR (Nujol) : 1660, 1680 $cm^{-1}$

Example 45

The following compounds were prepared in a similar manner to that of Example 28(2).

(1) (3R,6RS)-3-(1-Methylindol-3-yl)methyl-6-(3-pyridylmethyl)piperazine-2,5-dione
IR (Nujol) : 3200, 1670 $cm^{-1}$

(2) (3R,6R)-3-(1-Methylindol-3-yl)methyl-6-(4-pyridylmethyl)piperazine-2,5-dione
mp : 263-271°C

(3) (3R,6R)-3-Butyl-6-(1-naphthylmethyl)piperazine-2,5-dione
IR (Nujol) : 3190, 3040, 1670, 1660 $cm^{-1}$

(4) (3R,6R)-3-[(R)-1-(1-Ethylindol-3-yl)ethyl]-6-(2-pyridylmethyl)piperazine-2,5-dione
IR (Nujol) : 3200, 1675, 1660 $cm^{-1}$

(5) (3R,6R)-3-[(R)-1-(5-Chloro-1-methylindol-3-yl)-ethyl]-6-(2-pyridylmethyl)piperazine-2,5-dione
IR (Nujol) : 3210, 1680, 1660 $cm^{-1}$

(6) (3R,6R)-3-[(R)-1-(5-Methoxy-1-methylindol-3-yl)-ethyl]-6-(2-pyridylmethyl)piperazine-2,5-dione
IR ($CHCl_3$) : 3400, 1675 $cm^{-1}$

(7) (3R,6R)-3-[(R)-1-(1-Methylindol-3-yl)ethyl]-6-(1,3-thiazol-4-ylmethyl)piperazine-2,5-dione
IR ($CHCl_3$) : 3400, 1680 $cm^{-1}$

(8) (3R,6R)-3-[(R)-1-(5-Chloro-1-ethylindol-3-yl)-ethyl]-6-(2-pyridylmethyl)piperazine-2,5-dione
IR (Nujol) : 3210, 1675, 1655 $cm^{-1}$

(9) (3R,6R)-3-[(R)-1-(1-Propylindol-3-yl)ethyl]-6-(2-pyridylmetyl)piperazine-2,5-dione
IR (Nujol) : 1650 $cm^{-1}$

(10) (3R,6S)-3-[(R)-1-(1-Propylindol-3-yl)ethyl]-6-(2-pyridylmethyl)piperazine-2,5-dione
IR (Nujol) : 1670 $cm^{-1}$

(11) (3R,6R)-3-[(R)-1-(5-Fluoro-1-methylindol-3-yl)ethyl]-6-(2-pyridylmethyl)piperazine-2,5-dione
IR (Nujol) : 1665 $cm^{-1}$

(12) (3R,6S)-3-[(R)-1-(5-Fluoro-1-methylindol-3-yl)ethyl]-6-(2-pyridylmethyl)piperazine-2,5-dione
IR (Nujol) : 1670 $cm^{-1}$

(13) (3R,6S)-3-[(R)-1-(5-Chloro-1-ethylindol-3-yl)ethyl]-6-(2-pyridylmethyl)piperazine-2,5-dione
IR (Nujol) : 1670 $cm^{-1}$

(14) (3R,6R)-3-[(R)-1-(5-Chloro-1-ethylindol-3-yl)ethyl]-6-(4-thiazolylmethyl)piperazine-2,5-dione
IR (Nujol) : 1670 $cm^{-1}$

(15) (3R,6S)-3-[(R)-1-(5-Chloro-1-ethylindol-3-yl)ethyl]-6-(4-thiazolylmethyl)piperazine-2,5-dione
IR (Nujol) : 1675 $cm^{-1}$

(16) (3R,6R)-3-[(R)-1-(1-Butyl-5-fluoroindol-3-yl)ethyl]-6-(2-pyridylmethyl)piperazine-2,5-dione
IR (Nujol) : 1680 $cm^{-1}$

(17) (3R,6S)-3-[(R)-1-(1-Butyl-5-fluoroindol-3-yl)ethyl]-6-(2-pyridylmethyl)piperazine-2,5-dione
IR (Nujol) : 1665 $cm^{-1}$

(18) (3R,6R)-3-[(R)-1-(1-Butyl-5-fluoroindol-3-yl)ethyl]-6-(4-thiazolylmethyl)piperazine-2,5-dione
IR (Nujol) : 1670 $cm^{-1}$

(19) (3R,6R)-3-[(R)-1-(1-Butyl-5-chloro-2-methylindol-3-yl)-ethyl]-6-(2-pyridylmethyl)piperazine-2,5-dione
IR (Nujol) : 1680 $cm^{-1}$

(20) (3R,6S)-3-[(R)-1-(1-Butyl-5-chloro-2-methylindol-3-yl)-ethyl]-6-(2-pyridylmethyl)piperazine-2,5-dione
IR (Nujol) : 1660 $cm^{-1}$

(21) (3R,6R)-3-[(R)-1-(1-Butyl-5-chloro-2-methylindol-3-yl)-ethyl]-6-(4-thiazolylmethyl)piperazine-2,5-dione
IR (Nujol) : 1670 $cm^{-1}$

(22) (3R,6R)-3-[(R)-1-(5-Methyl-1-propylindol-3-yl)ethyl]-6-(2-pyridylmethyl)piperazine-2,5-dione
IR (Nujol) : 3200, 1665 $cm^{-1}$

(23) (3R,6S)-3-[(R)-1-(5-Methyl-1-propylindol-3-yl)ethyl]-6-(2-pyridylmethyl)piperazine-2,5-dione
IR (Nujol) : 3200, 1670 $cm^{-1}$

(24) (3R,6R)-3-[(R)-1-(5-Methyl-1-propylindol-3-yl)ethyl]-6-(4-thiazolylmethyl)piperazine-2,5-dione
IR (Nujol) : 3200, 1665 $cm^{-1}$

(25) (3R,6R)-3-[(R)-1-(2,5-Dimethyl-1-propylindol-3-yl)-ethyl]-6-(2-pyridylmethyl)piperazine-2,5-dione
IR (Nujol) : 3340, 3220, 3100, 1680, 1650 $cm^{-1}$

(26) (3R,6S)-3-[(R)-1-(2,5-Dimethyl-1-propylindol-3-yl)-ethyl]-6-(2-pyridylmethyl)piperazine-2,5-dione
IR (Nujol) : 3200, 1665 $cm^{-1}$

(27) (3R,6R)-3-[(R)-1-(1,2-Dimethylindol-3-yl)ethyl]-6-(2-pyridylmethyl)piperazine-2,5-dione
IR (Nujol) : 1675, 1650 $cm^{-1}$

(28) (3R,6S)-3-[(R)-1-(1,2-Dimethylindol-3-yl)ethyl]-6-(2-pyridylmethyl)piperazine-2,5-dione
IR (Nujol) : 1675 $cm^{-1}$

(29) (3R,6R)-3-[(R)-1-(1,5-Dimethylindol-3-yl)ethyl]-6-(2-pyridylmethyl)piperazine-2,5-dione
IR (Nujol) : 3200, 1650 $cm^{-1}$

(30) (3R,6S)-3-[(R)-1-(1,5-Dimethylindol-3-yl)ethyl]-6-(2-pyridylmethyl)piperazine-2,5-dione
IR (Nujol) : 3200, 1660 $cm^{-1}$

(31) (3R,6R)-3-[(R)-1-(5-Chloro-2-methyl-1-propylindol-3-yl)ethyl]-6-(2-pyridylmethyl)piperazine-2,5-dione
IR (Nujol) : 1660 $cm^{-1}$

(32) (3R,6S)-3-[(R)-1-(5-Chloro-2-methyl-1-propylindol-3-yl)ethyl]-6-(2-pyridylmethyl)piperazine-2,5-dione
IR (Nujol) : 1670 $cm^{-1}$

(33) (3R,6R)-3-[(R)-1-(5-Chloro-2-methyl-1-propylindol-3-yl)ethyl]-6-(4-thiazolylmethyl)piperazine-2,5-dione
IR (Nujol) : 1660 $cm^{-1}$

(34) (3R,6R)-3-[(R)-1-(1-Butyl-2-methylindol-3-yl)ethyl]-6-(4-thiazolylmethyl)piperazine-2,5-dione
IR ($CHCl_3$) : 1680 $cm^{-1}$

(35) (3R,6R)-3-[(R)-1-(1-Butyl-2-methylindol-3-yl)ethyl]-6-(2-pyridylmethyl)piperazine-2,5-dione
IR ($CHCl_3$) : 1677 $cm^{-1}$

(36) (3R,6S)-3-[(R)-1-(1-Butyl-2-methylindol-3-yl)ethyl]-6-(2-pyridylmethyl)piperazine-2,5-dione
IR ($CHCl_3$) : 1680 $cm^{-1}$

(37) (3R,6R)-3-[(R)-1-(1-Isobutylindol-3-yl)ethyl]-6-(2-pyridylmethyl)piperazine-2,5-dione
IR ($CHCl_3$) : 1678 $cm^{-1}$

(38) (3R,6S)-3-[(R)-1-(1-Isobutylindol-3-yl)ethyl]-6-(2-pyridylmethyl)piperazine-2,5-dione
IR ($CHCl_3$) : 1680 $cm^{-1}$

(39) (3R,6R)-3-[(R)-1-(1-Isobutylindol-3-yl)ethyl]-6-(4-thiazolylmethyl)piperazine-2,5-dione
IR ($CHCl_3$) : 1679 $cm^{-1}$

(40) (3R,6R)-3-[(R)-1-(1-Methyl-2-phenylindol-3-yl)-ethyl]-6-(2-pyridylmethyl)piperazine-2,5-dione
IR (Nujol) : 1680 $cm^{-1}$

(41) (3R,6S)-3-[(R)-1-(1-Methyl-2-phenylindol-3-yl)-ethyl]-6-(2-pyridylmethyl)piperazine-2,5-dione
IR (Nujol) : 1675 $cm^{-1}$

Example 46 (Preparation of granules or small granules)

| | |
|---|---|
| (3R,6R)-3-[(R)-1-(1-Methylindol-3-yl)ethyl]-6-(2-pyridylmethyl)piperazine-2,5-dione | 500(g) |
| Sucrose | 9000(g) |
| Hydroxypropylcellulose | 200(g) |
| Starch | 300(g) |

The above ingredients are blended and granulated in a conventional manner into granules or small granules.

We claim:

1. A piperazine compound of the formula:

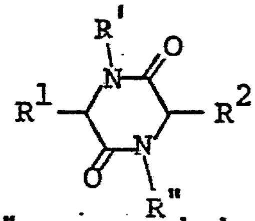

(I)

wherein R' and R" are each hydrogen or lower alkyl,

$R^1$ is 1-(1-methylindol-3-yl)ethyl,
1-(1-ethylindol-3-yl)ethyl,
1-(1-propylindol-3-yl)ethyl,
1-(1,2-dimethylindol-3-yl)ethyl,
1-(1-methyl-2-phenylindol-3-yl)ethyl,
1-(1,5-dimethylindol-3-yl)ethyl,
1-(5-methyl-1-propylindol-3-yl)ethyl,
1-(2,5-dimethyl-1-propylindol-3-yl)ethyl,
1-(1,2,5-trimethylindol-3-yl)ethyl
1-(1-butyl-2-methylindol-3-yl)ethyl,
1-(1-isobutylindol-3-yl)ethyl,
1-(1-methylindol-3-yl)propyl,
1-(1-ethyl-6-methylindol-3-yl)ethyl or
1-(1-ethyl-5-methylindol-3-yl)ethyl,

$R^2$ is 2-pyridylmethyl; or

$R^1$ is 1-methylindol-3-ylmethyl and

$R^2$ is 3-(or 4-)pyridylmethyl; or

$R^1$ is 1-naphthylmethyl and

$R^2$ is butyl or

$R^1$ is heterocyclic(lower)alkyl which may have one or more lower alkyl(s) on the heterocyclic ring and

$R^2$ is thiazolyl(lower)alkyl,
quinolyl(lower)alkyl,
tetahydropyridyl(lower)alkyl which may have lower alkyl on the tetrahydropyridine ring, or a group of the formula :

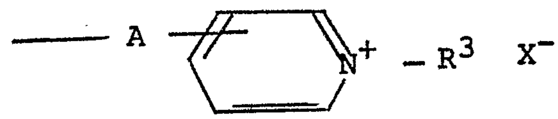

wherein A is lower alkylene,
$R^3$ is lower alkyl and
X is acid residue; or

$R^1$ is indolyl(lower)alkyl having lower alkyl and lower alkoxy on the indole ring, indolyl(lower)alkyl having one or more lower alkyl(s) and halogen on the indole ring, indolyl(lower)alkyl having lower alkyl and ar(lower)alkoxy on the indole ring or indolylar(lower)alkyl having lower alkyl on the indole ring and

$R^2$ is heterocyclic(lower)alkyl,

or pharmaceutically acceptable salt thereof.

2. A piperazine compound of claim 1, in which $R^1$, $R^2$, A, $R^3$ and X are each as defined above, and R' and R" are each hydrogen.

3. A piperazine compound of claim 2, which is (3R, 6R)-3-[(R)-1-(1-methylindol-3-yl)ethyl]-6-(2-pyridylmethyl)piperazine-2,5-dione or pharmaceutically acceptable salt thereof.

4. A process for preparing a piperazine compound of the formula:

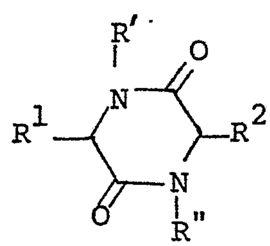

(I)

wherein R' and R" are each hydrogen or lower alkyl,
$R^1$ is 1-(1-methylindol-3-yl)ethyl,
1-(1-ethylindol-3-yl)ethyl,
1-(1-propylindol-3-yl)ethyl,
1-(1,2-dimethylindol-3-yl)ethyl,
1-(1-methyl-2-phenylindol-3-yl)ethyl,
1-(1,5-dimethylindol-3-yl)ethyl,
1-(5-methyl-1-propylindol-3-yl)ethyl,
1-(2,5-dimethyl-1-propylindol-3-yl)ethyl,
1-(1,2,5-trimethylindol-3-yl)ethyl
1-(1-butyl-2-methylindol-3-yl)ethyl,
1-(1-isobutylindol-3-yl)ethyl,
1-(1-methylindol-3-yl)propyl,
1-(1-ethyl-6-methylindol-3-yl)ethyl or
1-(1-ethyl-5-methylindol-3-yl)ethyl,
$R^2$ is 2-pyridylmethyl; or
$R^1$ is 1-methylindol-3-ylmethyl and
$R^2$ is 3-(or 4-)pyridylmethyl; or
$R^1$ is 1-naphthylmethyl and
$R^2$ is butyl or
$R^1$ is heterocyclic(lower)alkyl which may have one or more lower alkyl(s) on the heterocyclic ring and
$R^2$ is thiazolyl(lower)alkyl,
quinolyl(lower)alkyl,
tetahydropyridyl(lower)alkyl which may have lower alkyl on the tetrahydropyridine ring, or a group of the formula :

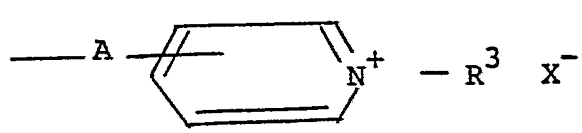

wherein A is lower alkylene,
$R^3$ is lower alkyl and
X is acid residue; or

$R^1$ is indolyl(lower)alkyl having lower alkyl and lower alkoxy on the indole ring, indolyl(lower)alkyl having one or more lower alkyl(s) and halogen on the indole ring, indolyl(lower)alkyl having lower alkyl and ar(lower)alkoxy on the indole ring or indolylar(lower)alkyl having lower alkyl on the indole ring and
$R^2$ is heterocyclic(lower)alkyl,
or salt thereof,
which comprises,
(a) subjecting a compound of the formula:

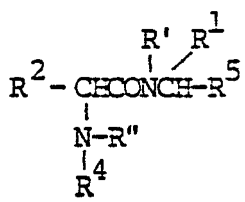 or 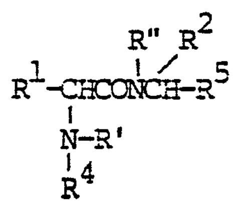

wherein $R^1$, $R^2$, $R'$ and $R''$ are each as defined above,
$R^4$ is hydrogen or amino-protective group and
$R^5$ is carboxy or a protected carboxy,
or their reactive derivative at the carboxy or amino group, or salt thereof,
to ring closure reaction to give a compound of the formula:

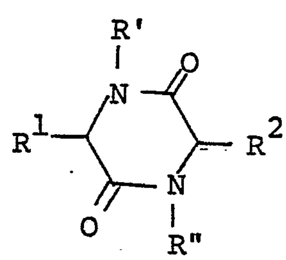

wherein $R^1$ , $R^2$, R', and R" are each as defined above or salt thereof; or

(b) reacting a compound of the formula:

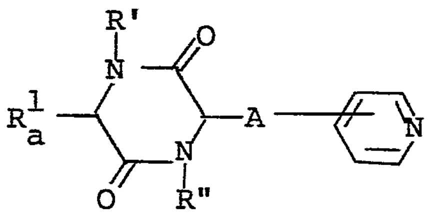

wherein A, R' and R" are each as defined above and

$R_a^1$ is heterocyclic(lower)alkyl which may have lower alkyl(s) on the heterocyclic ring,

or salt thereof

with a compound of the formula:

$$R^3X$$

wherein $R^3$ and X are each as defined above,

to give a compound of the formula:

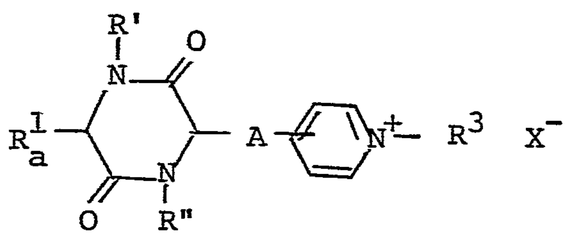

wherein $R_a^1$, A, $R^3$, R', R" and X are each as defined above;

(c) reducing a compound of the formula:

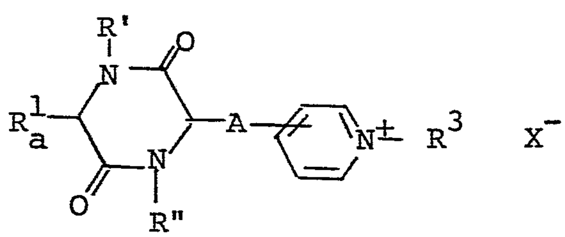

wherein $R_a^1$, A, $R^3$, R', R" and X are each as defined above, to give a compound of the formula:

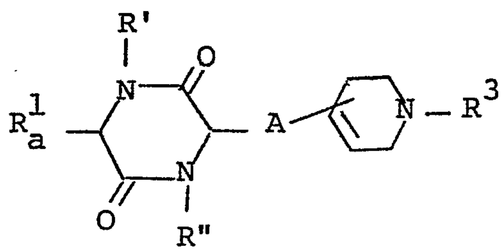

wherein $R_a^1$, A, $R^3$ R' and R" are each as defined above.

5. A pharmaceutical composition comprising, as an effective ingredient, a piperazine compound of the formula:

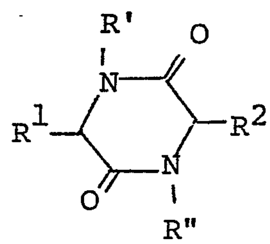

(I)

wherein R' and R" are each hydrogen or lower alkyl,

$R^1$ is 1-(1-methylindol-3-yl)ethyl,
1-(1-ethylindol-3-yl)ethyl,
1-(1-propylindol-3-yl)ethyl,
1-(1,2-dimethylindol-3-yl)ethyl,
1-(1-methyl-2-phenylindol-3-yl)ethyl,
1-(1,5-dimethylindol-3-yl)ethyl,
1-(5-methyl-1-propylindol-3-yl)ethyl,
1-(2,5-dimethyl-1-propylindol-3-yl)ethyl,
1-(1,2,5-trimethylindol-3-yl)ethyl
1-(1-butyl-2-methylindol-3-yl)ethyl,
1-(1-isobutylindol-3-yl)ethyl,
1-(1-methylindol-3-yl)propyl,
1-(1-ethyl-6-methylindol-3-yl)ethyl or
1-(1-ethyl-5-methylindol-3-yl)ethyl,

$R^2$ is 2-pyridylmethyl; or
$R^1$ is 1-methylindol-3-ylmethyl and
$R^2$ is 3-(or 4-)pyridylmethyl; or
$R^1$ is 1-naphthylmethyl and
$R^2$ is butyl or
$R^1$ is heterocyclic(lower)alkyl which may have one or more lower alkyl(s) on the heterocyclic ring and
$R^2$ is thiazolyl(lower)alkyl, quinolyl(lower)alkyl, tetahydropyridyl(lower)alkyl which may have lower alkyl on the tetrahydropyridine ring, or a group of the formula :

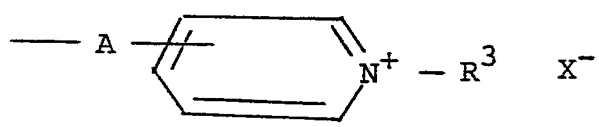

wherein A is lower alkylene,
$R^3$ is lower alkyl and
X is acid residue; or

$R^1$ is indolyl(lower)alkyl having lower alkyl and lower alkoxy on the indole ring, indolyl(lower)alkyl having one or more lower alkyl(s) and halogen on the indole ring, indolyl(lower)alkyl having lower alkyl and ar(lower)alkoxy on the indole ring or indolyl ar(lower)alkyl having lower alkyl on the indole ring and
$R^2$ is heterocyclic(lower)alkyl,
or pharmaceutically acceptable salt thereof, and pharmaceutically acceptable carrier(s).